(19) Europäisches Patentamt
European Patent Office
Office européen des brevets

(11) **EP 1 813 937 A1**

(12) **EUROPÄISCHE PATENTANMELDUNG**

(43) Veröffentlichungstag:
**01.08.2007  Patentblatt 2007/31**

(51) Int Cl.:
*G01N 27/30* (2006.01)     *G01N 33/487* (2006.01)
*G01N 27/04* (2006.01)     *C12Q 1/00* (2006.01)

(21) Anmeldenummer: **06001499.0**

(22) Anmeldetag: **25.01.2006**

(84) Benannte Vertragsstaaten:
**AT BE BG CH CY CZ DE DK EE ES FI FR GB GR
HU IE IS IT LI LT LU LV MC NL PL PT RO SE SI
SK TR**
Benannte Erstreckungsstaaten:
**AL BA HR MK YU**

(71) Anmelder:
• **Roche Diagnostics GmbH
68305 Mannheim (DE)**
Benannte Vertragsstaaten:
**DE**
• **F.HOFFMANN-LA ROCHE AG
4070 Basel (CH)**
Benannte Vertragsstaaten:
**AT BE BG CH CY CZ DK EE ES FI FR GB GR HU
IE IS IT LI LT LU LV MC NL PL PT RO SE SI SK TR**

(72) Erfinder:
• **Marquant, Michael
68309 Mannheim (DE)**
• **Zapf, Udo
67346 Speyer (DE)**
• **Bainczyk, Gregor
68199 Mannheim (DE)**
• **Unkrig, Volker
68526 Ladenburg (DE)**
• **Lungu, Mihail
67365 Schwegenheim (DE)**
• **Kotzan, Holger
68526 Ladenburg (DE)**

(74) Vertreter: **Pfeifer, Hans-Peter et al
Durm & Partner
Patentanwälte
Beiertheimer Allee 19
76137 Karlsruhe (DE)**

(54) **Elektrochemisches Biosensor-Analysesystem**

(57)  Elektrochemisches Biosensor-Analysesystem zur Analyse einer Probenflüssigkeit, insbesondere einer Körperflüssigkeit. Es umfaßt einen zur einmaligen Verwendung vorgesehenen Biosensor (2) mit einer Elektrodenstruktur (16) aus mindestens zwei Elektroden (17,18), einem die Elektrodenstruktur (16) überdeckenden, zur Aufnahme der Probenflüssigkeit geeigneten Testfeld und einer definierten Probenaufgabefläche, auf der Oberseite des Testfeldes, und ein Auswertegerät mit einer Auswerteeinheit zur Ermittlung der gewünschten Analysedaten.

Die Elektrodenstruktur ist auf einer von der Probenflüssigkeit benetzbaren effektiven Elektrodenstrukturfläche (23) hinsichtlich einer Widerstandsmessung an einer Flüssigkeitsschicht mit einer definierten Leitfähigkeit und Schichtdicke homogen verteilt. Das Testfeld ist so ausgebildet, daß ein Tropfen Probenflüssigkeit sich mindestens über eine Teilfläche der Probenaufgabefläche ausbreitet und eine entsprechende Teilfläche der effektiven Elektrodenstrukturfläche (23) benetzt wird. Das Auswertegerät weist eine Wechselstromwiderstandsmeßeinrichtung auf, um mittels einer Flächenkompensationsmessung einen Wechselstromwiderstandsmeßwert zwischen zwei Elektroden (17,18) der Elektrodenstruktur (16) zu messen, wobei der gemessene Wechselstromwiderstandsmeßwert bei der Ermittlung der Analysedaten als Maß für die von der Probenflüssigkeit benetzte Teilfläche der effektiven Elektrodenstrukturfläche (23) verwendet wird.

EP 1 813 937 A1

**Fig. 4**

**Beschreibung**

Die Erfindung betrifft eine elektrochemisches Biosensor-Analysesystem zur Analyse einer Probenflüssigkeit sowie einen entsprechenden Biosensor und ein mit dem System durchzuführendes Analyseverfahren. Die Analyse dient insbesondere medizinischen Zwecken, wobei die Probenflüssigkeit eine Körperflüssigkeit ist. Ein besonders wichtiges Anwendungsgebiet der Erfindung ist die Untersuchung sehr kleiner Probenmengen (weniger als 5 $\mu$l, vorzugsweise weniger als 2 $\mu$l und besonders bevorzugt weniger als 1 $\mu$l), die typischerweise durch einen Stich in die Haut gewonnen werden.

Zu dem Analysesystem gehören zwei Bestandteile, nämlich ein zur einmaligen Verwendung vorgesehener (disposibler) Biosensor und ein Auswertegerät.

Der Biosensor (der auch als "Analyseelement" oder "Testträger" bezeichnet wird) hat eine Tragschicht und eine auf der Tragschicht angeordnete Elektrodenstruktur mit mindestens zwei Elektroden. Auf der Tragschicht befindet sich ein die Elektrodenstruktur überdeckendes zur Aufnahme von Probenflüssigkeit geeignetes Testfeld, das ein Reagenzsystem enthält, dessen Reaktion mit der Probenflüssigkeit zu einer Änderung einer für das gewünschte analytische Ergebnis charakteristischen elektrischen Meßgröße führt, die mittels der Elektroden gemessen werden kann. Auf der von der Tragschicht abgewandten Oberseite des Testfeldes ist eine definierte Probenaufgabefläche vorgesehen.

Das Auswertegerät weist eine Einrichtung zum Positionieren eines Biosensors in einer Meßposition auf. Außerdem enthält es eine Meßeinrichtung, die zum Messen der Meßgröße an jeweils einem Biosensor geeignet ist, und eine Auswerteeinheit zur Ermittlung der gewünschten Analysedaten aus gemessenen Werten der Meßgröße.

Biosensor-Analysesysteme sind in zahlreichen Varianten vor allem zur Detektion (quantitativen oder qualitativen Bestimmung) unterschiedlicher Analyten gebräuchlich. Besonders große medizinische und wirtschaftliche Bedeutung hat die Bestimmung der Glucosekonzentration im Blut von Diabetikern. Andere wichtige Analyten sind Cholesterin und verschiedene Blutgerinnungsparameter. Das letztgenannte Beispiel zeigt, daß als Analyseparameter im Sinne der Erfindung nicht notwendigerweise die Konzentration einer Substanz in der Probenflüssigkeit zu verstehen ist, sondern daß sich die Erfindung auch auf andere (insbesondere auf medizinischem Gebiet) relevante Analyseparameter, wie hier die Blutgerinnungszeit, bezieht. Die Erfindung ist hinsichtlich des Analyseparameters nicht beschränkt.

Teilweise werden Biosensor-Analysesysteme in medizinischen Labors eingesetzt. Die Erfindung richtet sich jedoch insbesondere auf Anwendungsfälle, bei denen die Analyse durch den Patienten selbst durchgeführt wird, um seinen Gesundheitszustand laufend zu überwachen ("home-monitoring"). Für derartige Zwecke kommt es besonders auf eine einfache Handhabung an, weil nur dadurch sichergestellt werden kann, daß die erforderlichen Analysen von den Patients regelmäßig durchgeführt werden und die Genauigkeit des analytischen Ergebnisses nicht durch Handhabungsfehler beeinträchtigt wird. Außerdem müssen die Auswertegeräte möglichst klein, leicht und robust sein. Daneben ist die Erfindung auch für die sogenannte patientennahe Diagnostik ("near patient testing") in besonderem Maße geeignet.

Als Tragschicht bekannter Biosensoren werden meist längliche Kunststoffstreifen verwendet, jedoch gibt es auch andere Formen, beispielsweise näherungsweise quadratische Plättchen. Zu Beginn der Entwicklung waren vor allem teststreifenförmige Biosensoren gebräuchlich, deren Testfelder ein- oder mehrschichtig aus einem saugfähigen (porösen) Material, beispielsweise Papier oder porösem Kunststoff, hergestellt waren. Auf die Oberfläche des Testfeldes wurde ein Tropfen Probenflüssigkeit im Überschuß aufgetragen. Der Überschuß wurde abgewischt oder abgewaschen. Die Reaktion der in das Testfeld eindringenden Probenflüssigkeit mit dem darin enthaltenen (aus einem oder mehreren Bestandteilen bestehenden) Reagenzsystem führte zu einer Änderung der Farbe des Testfeldes, die mittels einer in dem zugehörigen Auswertegerät enthaltenen Photometereinrichtung ausgewertet wurde.

Auch bei elektrochemischen Analysesystemen, auf die sich die vorliegende Erfindung richtet, waren in der Anfangszeit der Entwicklung Gestaltungen des Biosensors mit einem porösen Testfeld gebräuchlich, auf dessen Oberseite die Probenflüssigkeit aufgegeben wurde. Dies ist beispielsweise aus dem 1989 angemeldeten US-Patent 5,243,516 bekannt. Später wurden Kapillar-Biosensoren vorgeschlagen, die einen Kapillarkanal aufweisen, in dem die Probenflüssigkeit von einer Eintrittsöffnung durch Kapillarwirkung in eine Reaktionszone transportiert wird, in der sich das Reagenzsystem und die Elektroden befinden. Frühe Varianten elektrochemischer Kapillar-Biosensoren sind beispielsweise aus der EP 0170375 A2 und dem US-Patent 5,120,420 bekannt. In der Folgezeit wurde dieses Konstruktionsprinzip, insbesondere für elektrochemische Biosensoren, nahezu ausschließlich angewandt, wobei als vorteilhaft insbesondere die Tatsache angesehen wurde, daß ein Kapillar-Biosensor eine definierte Probenmenge aufnimmt, die dem Volumen des Kapillarkanals einschließlich der Reaktionszone entspricht. Diese Eigenschaft wird auch als "self-metering" bezeichnet. Daneben werden Kapillar-Biosensoren hinsichtlich ihrer Handhabung als vorteilhaft angesehen, weil es genügt, einen beispielsweise an der Fingerkuppe erzeugten Blutstropfen in Kontakt mit der Eintrittsöffnung des Kapillarkanals zu bringen, wobei die Probenflüssigkeit rasch und sicher aufgesaugt wird. Neuere Kapillar-Biosensoren sind beispielsweise in dem US-Patent 6,645,359 und in der WO 2004/068138 beschrieben.

Auf dieser Grundlage befaßt sich die Erfindung mit dem technischen Problem, ein Biosensor-Analysesystem zur Verfügung zu stellen, das für eine Mehrzahl unterschiedlicher Anwendungsfälle eine erleichterte Handhabung ermöglicht. Dieses Problem wird erfindungsgemäß gelöst durch eine Analysesystem der eingangs bezeichneten Art, bei dem die Elektrodenstruktur auf einer von der Probenflüssigkeit benetzbaren effektiven Elektrodenstrukturfläche hinsichtlich einer Widerstandsmessung an einer Flüssigkeitsschicht mit einer definierten Leitfähigkeit und Schichtdicke homogen verteilt ist, das Testfeld so ausgebildet ist, daß ein auf die Probenaufgabefläche aufgegebener Tropfen Probenflüssigkeit sich mindestens über eine Teilfläche der Probenaufgabefläche ausbreitet und eine entsprechende Teilfläche der effektiven Elektrodenstrukturfläche benetzt wird, und das Auswertegerät eine Wechselstromwiderstandsmeßeinrichtung aufweist, um einen Wechselstromwiderstandsmeßwert zwischen zwei Elektroden der Elektrodenstruktur als Flächenkompensationsmessung zu messen, wobei der gemessene Wechselstromwiderstandsmeßwert bei der Ermittlung der Analysedaten als Maß für die von der Probenflüssigkeit benetzte Teilfläche der effektiven Elektrodenstrukturfläche verwendet wird.

Die Erfinder haben erkannt, daß Kapillar-Biosensoren in vielen Fällen nur eingeschränkt anwendbar sind oder statt der erhofften Erleichterung Schwierigkeiten hinsichtlich der Herstellung und/oder Handhabung verursachen. Dies gilt beispielsweise in folgenden Fällen:

a) Vielfach ist es vorteilhaft, die Reaktionszone des Biosensors zu erwärmen, insbesondere um die Reaktion zu beschleunigen. Dadurch wird unter anderem die Möglichkeit geschaffen, Reaktanten (insbesondere Enzyme) zu verwenden, die besonders spezifisch sind, aber bei Raumtemperatur zu langsam reagieren. Die Erwärmung führt zu einer Verbesserung der Analysequalität und/oder zu einer Verkürzung der erforderlichen Reaktionszeit. Besonders, bevorzugt ist die Thermostatisierung der Reaktionszone mittels einer Temperaturmeßeinrichtung und einer in das Auswertegerät integrierten Thermostatisierungselektronik.
Die Heizeinrichtung und gegebenenfalls auch die Temperaturmeßeinrichtung befindet sich vorteilhaft im Inneren des Auswertegerätes. Die Zuführung der Probe zu üblichen Kapillar-Biosensoren erfolgt hingegen außerhalb des Gerätes. Dies bedingt einen langen Kapillarkanal und folglich ein großes Probevolumen. Um dieses Problem mindestens hinsichtlich der Temperaturmessung zu überwinden, wurden spezielle Temperaturmeßverfahren (US-Patent 6,880,968) und Korrekturverfahren (WO 2004/090533), die ohne Temperaturmessung eine Temperaturkompensation ermöglichen, vorgeschlagen. Diese Vorschläge ermöglichen jedoch keine Beheizung der Meßzone. Auf der Grundlage der vorliegenden Erfindung ist es hingegen möglich, den Biosensor in dem Analysegerät so zu positionieren, daß sich die durch das Testfeld definierte Reaktionszone an einer zentralen Position des Auswertegerätes in unmittelbarem Kontakt zu einer Heizeinrichtung befindet und dennoch die Kontaktierung des Biosensors mit der Probe auf sehr einfache Weise erfolgen kann.

b) Analysesysteme, die mit bandförmig aneinandergereihten Biosensoren arbeiten, sind in vielen Fällen vorteilhaft, weil sie auf einfache Weise die Magazinierung einer Vielzahl von Biosensoren, sowie deren Transport und Positionierung in dem Auswertegerät ermöglichen. Es ist zwar möglich, Kapillar-Biosensoren in Form von Sensorbändern herzustellen und in entsprechende Systeme zu integrieren (WO 2004/030822 A1). Herstellung und Handhabung solcher Kapillar-Biosensor-Bänder sind jedoch auf der Grundlage der vorliegenden Erfindung einfacher möglich.

c) Ähnliches gilt für Mehrfach-Biosensoren, die auf einer flachen, beispielsweise etwa scheckkartenförmigen, Platte eine Mehrzahl von Testfeldern aufweisen. Solche Mehrfach-Testkarten sind beispielsweise (als sogenannte "Day-Packs") für Anwendungsfälle geeignet, bei denen der Benutzer täglich eine bestimmte Anzahl von Analysen durchführt, wobei die hierfür erforderlichen Biosensoren auf einer gemeinsamen Tragplatte positioniert sind. Auch in diesem Fall ist es sehr schwierig und aufwendig, eine Kapillar-Biosensorkonstruktion zu verwenden.

d) Auf dem Gebiet der patientennahen Diagnostik besteht ein wichtiger Vorteil der Erfindung darin, daß die Probe auch aus einer Spritze problemlos auf das Probenaufgabefeld aufgetragen werden kann. Bei Kapillarsensoren besteht diese Möglichkeit der Probenzuführung nicht oder nur mit einer sehr komplizierten Handhabung.

Ein wesentlicher Vorteil der Erfindung besteht darin, daß das Testfeld verhältnismäßig groß sein kann, aber nicht vollständig mit der Probenflüssigkeit benetzt werden muß. Bevorzugt entspricht die Größe der Probenaufgabefläche einem Kreis mit mindestens 8 mm, bevorzugt mindestens 10 mm und besonders bevorzugt mindestens 12 mm Durchmesser. Die bevorzugte Mindestfläche, die nicht kreisförmig sein muß, beträgt somit etwa 50 mm$^2$, besonders bevorzugt etwa 80 mm$^2$ und noch weiter bevorzugt etwa 115 mm$^2$.

Bei bekannten Systemen würde ein Meßfehler verursacht, wenn das Testfeld und damit die mit der Probenaufgabefläche kongruente Teilfläche der Elektrodenstruktur nicht vollständig benetzt wird. Im Rahmen der Erfindung erfolgt diesbezüglich eine Kompensation durch eine zusätzliche Messung ("Flächenkompensationsmessung") des elektrischen Wechselstromwiderstandes zwischen zwei Elektroden, wobei der gemessene Wechselstromwiderstandsmeßwert als Maß für die von der Probenflüssigkeit benetzte Teilfläche der Elektrodenfläche verwendet wird. Durch die Messung mit Wechselstrom wird erreicht, daß der Meßwert nicht von Transportvorgängen an der Elektrode beeinflußt wird. Vorzugsweise wird ein Wechselstrom mit einer Frequenz zwischen 1 kHz und 25 kHz verwendet, wobei Frequenzen zwischen 2 kHz und 10 kHz besondere bevorzugt sind.

Die Genauigkeit der Kompensation hängt davon ab, daß eine für diesen Zweck geeignete Elektrodenstruktur gewählt wird. Sie muß insbesondere auf der maximal mit der Probenflüssigkeit benetzbaren Teilfläche der Elektrodenstruktur, die hier als "effektive Elektrodenstrukturfläche" bezeichnet wird, hinsichtlich einer Wechselstrom-Widerstandsmessung an einer Flüssigkeitsschicht mit einer definierten Leitfähigkeit und Schichtdicke homogen sein. Dies bedeutet, daß bei Benetzung eines Teilbereiches der effektiven Elektrodenstrukturfläche mit einer Flüssigkeit, die eine definierte Schichtdicke und elektrische Leitfähigkeit hat, ein von der Lokalisierung des benetzten Teilbereiches unabhängiger, zu der Größe des benetzten Teilbereiches proportionaler Wechselstrom-Leitwert resultiert. Dieses Erfordernis wird nachfolgend als "Elektrodenstruktur -Homogenitätsbedingung" bezeichnet.

Um dies möglichst gut zu gewährleisten, sollten die Leiterbahnen, die die Elektrodenstruktur bilden, sehr fein und auf der effektiven Elektrodenstrukturfläche gleichmäßig (homogen) verteilt sein. Im Prinzip könnte diese Bedingung optimal mittels einer sehr großen Zahl von sehr dicht beieinander angeordneten kleinen Elektrodenpaaren erfüllt werden. In der Praxis stellen sich hierbei allerdings erhebliche Probleme hinsichtlich des elektrischen Anschlusses der Elektroden. Es wären mehrere Leiterbahnebenen erforderlich. Derartige Konstruktionen sind in der Elektronik zwar nicht ungebräuchlich, bedingen jedoch einen erheblichen Aufwand.

Bevorzugt ist deshalb eine Elektrodenstruktur mit zwei Elektroden, die jeweils kammartig mit einem Primärleiter und davon abzweigenden quer zu der Richtung des Primärleiters verlaufenden Sekundärleitern ausgebildet sind, wobei die Sekundärleiter der beiden Elektroden alternierend ineinandergreifen ("interdigitizing electrodes"). Vorzugsweise sind die Elektroden nicht funktionsdifferenziert, d.h. sie stimmen hinsichtlich Material und Anordnung so überein, daß sie wahlweise als Arbeits- und Gegenelektrode verwendet werden können.

Obwohl Ausführungsformen mit nur zwei Elektroden, die sowohl für die Analysemessung als auch für die Flächenkompensationsmessung verwendet werden, in der Regel vorteilhaft sind, ist die Erfindung hierauf nicht beschränkt. Grundsätzlich, wenn auch mit höherem Aufwand, sind auch Ausgestaltungen mit drei oder mehr Elektroden möglich. Es besteht auch die Möglichkeit, unterschiedliche Elektroden für die Analysemessung und für die Flächenkompensationsmessung einzusetzen.

Das Testfeld kann sehr unterschiedlich gestaltet sein. Es kann aus einer Schicht oder mehreren Schichten eines Materials bestehen, das geeignet ist, die Probenflüssigkeit aufzunehmen, wobei dieser Flüssigkeitsaufnahme unterschiedliche Mechanismen zugrunde liegen können. Insbesondere kann eine Porosität der für das Testfeld verwendeten Materialien und/oder einer Reaktion der Probenflüssigkeit mit dem Testfeldmaterial Ursache der Aufnahme der Probenflüssigkeit in das Testfeld sein. Vorzugsweise ist mindestens eine Teilschicht des Testfeldmaterials quellfähig, wobei Ausführungsformen besonders bevorzugt sind, bei denen die Dicke des Testfeldes (und damit dessen Volumen) durch den Quellvorgang bei der Aufnahme der Probenflüssigkeit mindestens auf das doppelte, vorzugsweise mindestens auf das dreifache, zunimmt.

Im Sinne der hier gegebenen Erläuterung wird nur derjenige Teil des Testfeldmaterials, der sich unter der Probenaufgabefläche befindet, als "Testfeld" bezeichnet. In der Regel erstreckt sich das Testfeldmaterial aus Produktionsgründen über eine größere Teilfläche der Biosensor-Tragschicht, wobei jedoch die definierte Probenaufgabefläche derartig begrenzt ist, daß außerhalb aufgegebene Probe nicht in das Testfeld eindringt. Bevorzugt ist der Biosensor so gestaltet, daß maximal das unter der Probenaufgabefläche befindliche (mit dieser kongruente) Testfeld von der Probenflüssigkeit benetzt werden kann.

Ein auf die Probenaufgabefläche aufgegebener Tropfen Probenflüssigkeit breitet sich über eine Teilfläche der Probenaufgabefläche aus und benetzt eine mit dieser Teilfläche kongruente Teilfläche der Elektrodenstrukturfläche. Je nach Größe des aufgegebenen Tropfens Probenflüssigkeit kann der Flächenanteil der benetzten Teilfläche an der gesamten effektiven Elektrodenstrukturfläche zwischen wenigen Prozent und 100 % liegen.

Die Flüssigkeitstransporteigenschaften des Testfeldes und der Meßalgorithmus sind so aufeinander abgestimmt, daß sich die benetzte Fläche der Elektrodenstruktur während der Analysemessung und der Flächenkompensationsmessung nicht wesentlich (d.h. in einem die gewünschte Meßgenauigkeit beeinträchtigenden Ausmaß) ändert. Diese Bedingung kann mit üblichen Testfeldmaterialien relativ einfach erfüllt werden, insbesondere weil die Analysemessung bei vielen gebräuchlichen Meßverfahren in sehr kurzer Zeit erfolgen und die Flächenkompensationsmessung simultan mit - oder in engem zeitlichen Abstand zu - der Analysemessung durchgeführt werden kann.

Die Erfindung wird nachfolgend anhand von in den Figuren dargestellten Ausführungsbeispielen näher erläutert. Die dargestellten Besonderheiten können einzeln oder in Kombination verwendet werden, um bevorzugte Ausgestaltungen der Erfindung zu schaffen. Es zeigen:

Fig. 1       eine perspektivische Darstellung der Bestandteile eines erfindungsgemäßen Analysesystems;

Fig. 2     eine Darstellung entsprechend Figur 1 mit in der Halterung des Auswertegerätes positioniertem Biosensor;

Fig. 3     eine Aufsicht auf einen erfindungsgemäßen Biosensor;

Fig. 4     eine Aufsicht auf die Elektrodenstruktur eines erfindungsgemäßen Biosensors;

Fig. 5     eines vergrößerten, nicht maßstäbliche, schematische Darstellung eines Querschnitts längs der Linie V-V von Figur 3;

Fig. 6     ein Prinzipschaltbild einer für die Erfindung geeigneten Elektronikeinheit;

Fig. 7     eine perspektivische Prinzipdarstellung von drei Phasen des Eindringens einer Probenflüssigkeit in ein Testfeld;

Fig. 8     die zeitliche Änderung von zwei während des Eindringens der Probenflüssigkeit in das Testfeld gewonnenen Meßsignalen;

Fig. 9     die zeitliche Änderung von Meßsignalen, einschließlich einer Flächenkompensationsmessung und einer Analysemessung;

Fig. 10    eine perspektivische Darstellung eines erfindungsgemäßen Analysesystems mit einer Mehrfach-Testkarte;

Fig. 11    eine Aufsicht auf eine für das System gemäß Fig. 10 geeignete Elektrodenstruktur;

Fig. 12    eine perspektivische Explosionsdarstellung der Konstruktion einer Mehrfach-Testkarte für ein System gemäß Figur 10;

Fig. 13    eine 4-Phasen-Prinzipdarstellung zur Erläuterung des Herstellungsprozesses eines Sensorbandes.

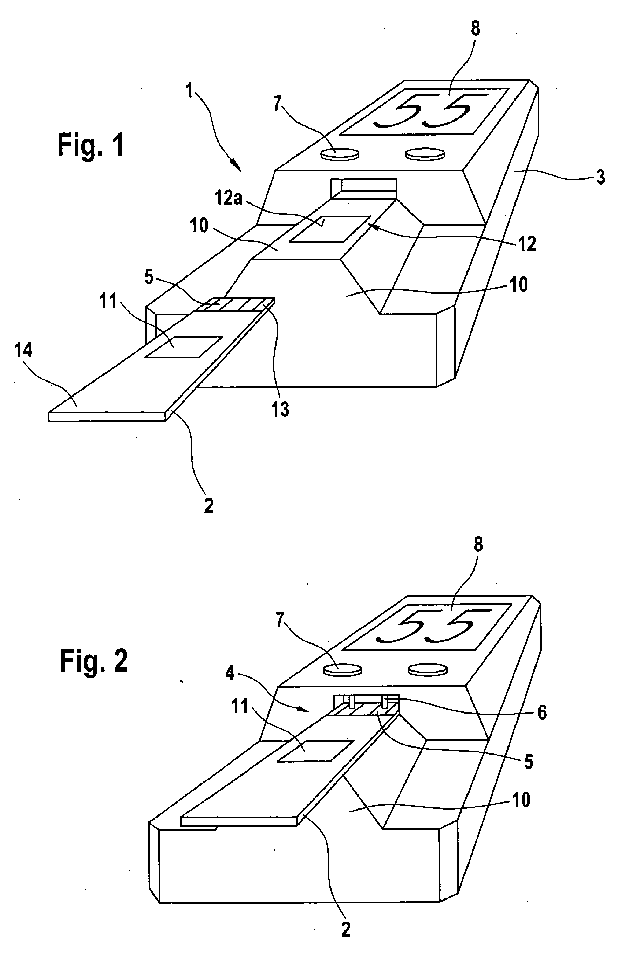

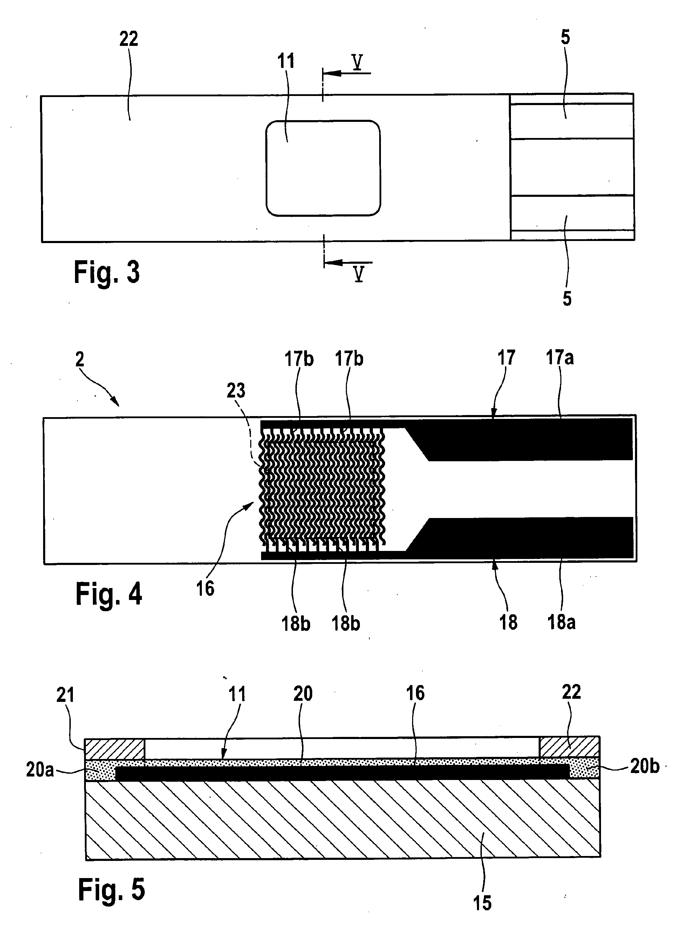

Das in den Figuren 1 und 2 dargestellte Analysesystem 1 besteht aus zwei aufeinander abgestimmten Systembestandteilen, nämlich einem Biosensor 2 und einem Auswertegerät 3. Das Auswertegerät 3 hat eine Halterung 4 zum Positionieren eines Biosensors 2 in einer in Figur 2 dargestellten Meßposition. Wenn sich der Biosensor 2 in der Meßposition befindet, besteht mittels Sensorkontakten 5 und mit diesen kooperierenden Gerätekontakten 6 eine elektrische Verbindung zwischen seinen Elektroden und einer Elektronikeinheit des Auswertegerätes. Das Auswertegerät 3 hat Bedienungstasten 7 und ein Display 8. Insoweit ist die Konstruktion konventionell und muß nicht näher erläutert werden.
Eine Besonderheit der dargestellten bevorzugten Ausführungsform besteht darin, daß ein in der Halterung 4 positionierter Biosensor 2 von einem Biosensorlager 10 unterstützt wird, das gegenüber den benachbarten Bauteilen des Auswertegerätes 3 (zumindest in der Umgebung der Probenaufgabefläche 11 eines in der Meßposition befindlichen Biosensors 2) erhaben ist. Der Biosensor 2 ist als langgestreckter Teststreifen ausgebildet. An seinem zum Einführen in die Halterung 4 des Auswertegerätes 3 vorgesehenen Einführungsende 13 befinden sich die Sensorkontakte 5. Das gegenüberliegende Ende des Biosensors 2 wird als Handhabungsende 14 bezeichnet. Es ragt über das Biosensorlager 10 hinaus. Durch diese Maßnahmen ergibt sich eine leichte Handhabung beim Einführen und Herausnehmen des Biosensors 2 und auch beim Aufgeben eines Tropfens Probenflüssigkeit auf die Probenaufgabefläche 11, wobei das Risiko einer Kontamination des Gerätes gering ist.
Die dargestellte bevorzugte Ausführungsform des Auswertegerätes 3 weist eine Heizeinrichtung 12 auf, die derartig positioniert ist, daß das unter der Probenaufgabefläche 11 befindliche Testfeld eines in der Meßposition gehaltenen Biosensors 2 gleichmäßig beheizt werden kann. Im dargestellten Fall dient hierzu eine Heizfläche 12a, die sich unter der Probenaufgabefläche 11 eines in der Meßposition positionierten Biosensors 2 erstreckt.
Einzelheiten eines bevorzugten Biosensors 2 sind in den Figuren 3 bis 5 dargestellt. Er hat eine Tragschicht 15 aus Kunststoff und eine auf der Tragschicht 15 angeordnete Elektrodenstruktur 16 mit zwei Elektroden 17,18. Im dargestellten Fall sind die Elektroden jeweils kammartig mit einem Primärleiter 17a,18a und davon abzweigenden, quer zu der Richtung des jeweiligen Primärleiters verlaufenden, Sekundärleitern 17b,18b dargestellt, wobei die Sekundärleiter 17b,18b der beiden Elektroden 17,18 alternierend ineinandergreifen.
Die Elektrodenstruktur 16 ist von einem das Reagenzsystem des Biosensors enthaltenden Testfeld 20 überdeckt, dessen Oberseite die Probenaufgabefläche 11 bildet. Die Probenaufgabefläche 11 ist mittels einer Maskierungsschicht 22 derartig begrenzt, daß Probenflüssigkeit nur über die Probenaufgabefläche 11 in das Testfeld 20 eindringen kann.
Das Testfeld 11 ist sehr dünn. Vorzugsweise besteht es aus einem quellfähigen Material, dessen Volumen sich, wie bereits dargelegt, bei der Aufnahme von Wasser aus der Probenflüssigkeit erheblich vergrö-ßert. Die Testfelddicke beträgt vorzugsweise maximal 150 $\mu$m, bevorzugt maximal 120 $\mu$m und besonders bevorzugt maximal 80 $\mu$m, wobei sich diese Maßangaben im Falle eines quellfähigen Materials auf die Naßschichtdikke des Testfeldes, also dessen nach der Flüssigkeitsaufnahme resultierende Dicke, beziehen. Die Naßschichtdicke ist für das erforderliche Probenvolumen entscheidend.

Aufgrund der geringen Dicke des Testfeldes findet - bei Verwendung üblicher Testfeldmaterialien - nur ein sehr geringfügiger Flüssigkeitstransport innerhalb des Testfeldes parallel zu der Testfeldoberfläche statt. Demzufolge kann praktisch nur die mit der Probenaufgabefläche 11 kongruente (deckungsgleich fluchtende) Teilfläche der Elektrodenstruktur 16 von der Probenflüssigkeit benetzt werden. Diese Teilfläche, die in Figur 4 mit einer gestrichelten Linie umgrenzt ist, wird als effektive Elektrodenstrukturfläche 23 bezeichnet. Auf diese effektive Elektrodenstrukturfläche 23 bezieht sich die oben erläuterte Elektrodenstruktur-Homogenitätsbedingung. Diese Bedingung wird dadurch erfüllt, daß die Elektroden innerhalb der effektiven Elektrodenstrukturfläche aus einer Vielzahl sehr kleiner Leiterbahnelemente bestehen, die im dargestellten Fall durch die Sekundärleiter 17b,18b gebildet werden. Es sind jedoch auch andere Gestaltungen möglich.

Der Abstand zwischen den Leiterbahnen, die die Elektrodenstruktur bilden, sollte sehr klein sein. Vorzugsweise liegt der durchschnittliche Leiterbahnenabstand auf der effektiven Elektrodenstrukturfläche 23 bei höchstens 50 μm, vorzugsweise höchstens 30 μm und besonders bevorzugt höchstens 10 μm. Auch die Breite der Leiterbahnen sollte sehr klein sein, wobei aber im Hinblick auf die Meßgenauigkeit eine ausreichende Mindestbreite vorteilhaft ist. Vorzugsweise beträgt die durchschnittliche Breite der Leiterbahnen auf der effektiven Elektrodenstrukturfläche mindestens 10 μm und vorzugsweise mindestens 30 μm. Andererseits sollte die durchschnittliche Leiterbahnbreite höchstens 150 μm, bevorzugt maximal 100 μm betragen.

Gemäß einer weiteren bevorzugten Maßnahme ändert sich die Richtung, in der die Sekundärleiter 17b,18b verlaufen, über deren innerhalb der effektiven Elektrodenstrukturfläche verlaufende Länge mehrfach. Im Rahmen der Erfindung wurde festgestellt, daß dadurch die Widerstandsmessung-Homogenitätsbedingung besser erfüllt wird. Statt der in Figur 4 dargestellten wellenförmigen Form können auch andere Gestaltungen mit einer Vielzahl von Richtungsänderungen, beispielsweise zickzack-förmig, gewählt werden.

Elektrodenstrukturen, die sich für die vorliegende Erfindung eignen und zur Herstellung solcher Strukturen bevorzugte Verfahren sind zu anderen Zwecken bereits in der Biosensor-Technik vorgeschlagen worden. Diesbezüglich wird auf das bereits erwähnte US-Patent 6,645,359 Bezug genommen und dessen Inhalt durch die Bezugnahme zum Inhalt der vorliegenden Anmeldung gemacht wird. Dieser Druckschrift lassen sich auch nähere Angaben über für die Tragschicht 15 geeignete Materialien entnehmen.

Erfindungsgemäße Biosensoren können einfach und ökonomisch hergestellt werden. Vorzugsweise wird auf einem Band des Materials, aus dem die Tragschicht 15 besteht, das nachfolgend als "Produktionsband" bezeichnet wird und dessen Breite der Länge der späteren Testträger entspricht, eine Vielzahl von nebeneinander angeordneten Elektrodenstrukturen 16 erzeugt. Dies geschieht vorzugsweise durch selektives Entfernen einer zunächst (z.B. durch Sputtern oder Bedampfen) flächig aufgetragenen Schicht eines geeigneten Elektrodenmaterials (beispielsweise Gold). Zum selektiven Entfernen der nicht gewünschten Teile der leitenden Beschichtung kann insbesondere das Laserablations-Verfahren eingesetzt werden. Es sind jedoch auch andere, insbesondere photolithographische, Verfahren geeignet. Figur 4 zeigt den einem einzelnen Biosensor entsprechenden Teil des Produktionsbandes nach der Ausbildung der Elektrodenstruktur.

Nach der Bildung der Elektrodenstruktur wird auf das Produktionsband das Testfeldmaterial mindestens in einer solchen Breite aufgetragen, daß die effektive Elektrodenstrukturfläche 23 vollständig und gleichmäßig bedeckt ist. In der Praxis erstreckt sich das Testfeldmaterial über, das Testfeld 20 (Figur 5) hinaus in Randbereiche 20a und 20b, die außerhalb der Probenaufgabefläche 11 liegen.

Der Auftrag des Testfeldmaterials erfolgt vorzugsweise mittels einer geeigneten Testschichtmasse, die im flüssigen oder pastösen Zustand aufgetragen wird und auf der Tragschicht 15 durch Erstarren und/oder Trocknen fest wird, wobei sie mindestens die effektive Elektrodenstrukturfläche überdeckt.

Danach wird die Maskierungsschicht 22 aufgebracht, die im Bereich der Probenaufgabefläche 11 und der Sensorkontakte 5 entsprechende Aussparungen ("Fenster") enthält. Sie besteht aus einem elektrisch isolierenden und vorzugsweise hydrophoben Kunststoffmaterial. Geeignet ist beispielsweise eine einseitig klebende Kunststofffolie 21. Die Maskierungsschicht muß jedoch nicht notwendigerweise folienförmig aufgebracht, sondern kann auch auf andere Weise, beispielsweise durch selektives Beschichten mit einer entsprechenden Masse oder Flüssigkeit erzeugt werden.

Das Testfeld 20 enthält bevorzugt alle Reagenzien, die für die gewünschte Analyse notwendig sind. Vorteilhaft ist es so ausgebildet, daß sich eine auf die Probenaufgabefläche 11 aufgegebene Probenflüssigkeit zunächst rasch und gleichmäßig auf der Oberfläche ausbreitet und dann senkrecht zu der Probenaufgabefläche in das Innere des Testfeldes eindringt. Wie erwähnt, kann das Testfeld aus mehreren unterschiedlichen Schichten bestehen. Bevorzugt ist jedoch eine einschichtige, homogene Testfeldstruktur.

Gemäß einer weiteren bevorzugten Ausführungsform enthält das Testfeld 20 Separationsmittel zur Abtrennung korpuskulärer Bestandteile aus der Probenflüssigkeit. Dadurch wird erreicht, daß im wesentlichen nur Plasma zu der Elektrodenstruktur 16 gelangt und diese benetzt, wobei "im wesentlichen" so zu verstehen ist, daß die korpuskulären Bestandteile so gut abgetrennt werden, daß die Meßgenauigkeit nicht in einem für den jeweiligen Test störenden Ausmaß beeinträchtigt wird. Diese Aussage bezieht sich auf den Meßzeitpunkt.

Selbstverständlich ist es irrelevant, wenn korpuskuläre Bestandteile zu der Elektrodenstruktur 16 vordringen, nachdem die für die Analyse erforderlichen Messungen durchgeführt sind.

Gemäß einer besonders bevorzugten Ausführungsform enthält das Testfeld 20 folgende Komponenten:

- Feste Mikropartikel, beispielsweise aus $SiO_2$. Sie bilden eine Grundstruktur, die vor allem flüssigkeitsausbreitende Eigenschaften hat.

- Quellmittel. Geeignet sind beispielsweise Zelluloseprodukte. Sie sorgen dafür, daß die Testschicht (vorzugsweise mindestens in dem weiter oben erläuterten Umfang) quillt.

- Lösliche Bestandteile, insbesondere Reagenzien (Enzym, Mediator, Puffer). Wenn diese Bestandteile beim Eindringen der Probenflüssigkeit gelöst werden, entstehen Mikrokapillaren, durch die die Flüssigkeit in die Tiefe der Schicht eindringt.

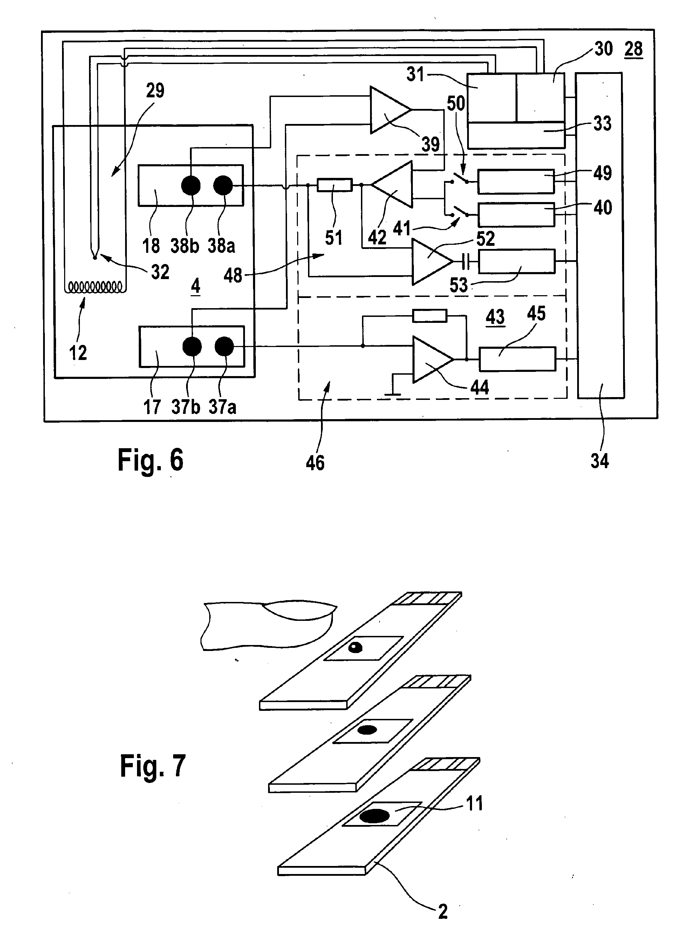

Die in Figur 6 in Form eines Prinzipschaltbildes dargestellte Elektronikeinheit eines für die Erfindung geeigneten Auswertegerätes enthält folgende Komponenten:

- Eine Heizungselektronik 30, durch die die im Bereich der Biosensor-Halterung 4 vorgesehene Heizeinrichtung 12 angesteuert und vorzugsweise thermostatisch geregelt wird.

- Eine Temperaturmeßeinrichtung 29, die mittels eines Meßsensors 32 (beispielsweise eines Thermistors) und einer Temperaturmeßelektro- , nik 31 die Temperatur des Testfeldes mißt.

- Eine Thermostatisierungselektronik 33, um das Testfeld 11 mittels der Temperaturmeßeinrichtung 29 und der Heizeinrichtung 12 auf eine gewünschte Solltemperatur zu thermostatisieren.

Diese Komponenten sind konventionell ausgebildet und müssen nicht näher erläutert werden.

Die Elektronikeinheit 28 schließt weiterhin eine mikroprozessorgesteuerte Kontroll- und Auswerteeinheit 34 ein, die die übrigen Komponenten der Elektronikeinheit 28 kontrolliert und aus den gewonnenen Meßdaten die gewünschten analytischen Ergebnisse oder sonstigen Informationen ermittelt.

Die dargestellte Elektronikeinheit 28 eignet sich für amperometrische Analysesysteme, bei denen die aus der Reaktion der Probenflüssigkeit mit dem Reagenzsystem resultierende charakteristische Meßgröße ein elektrischer Strom ist, der gemessen wird, wenn eine definierte Gleichspannung an die Elektroden angelegt wird. Dieses für die vorliegende Erfindung bevorzugte Testprinzip ist bekannt und in zahlreichen Publikationen beschrieben. Nähere Informationen können beispielsweise den bereits zitierten Dokumenten EP 0170375, US 5,120,420, WO 2004/090533 und US 6,645,359 sowie den darin zitierten weiteren Schriften entnommen werden.

Bei der in Figur 6 dargestellten Ausführungsform dienen jeweils zwei Kontakte dazu, die Arbeitselektrode 17 und die Gegenelektrode 18 an die Elektronikeinheit 28 anzuschließen. Dabei dienen die mit 37a und 38a bezeichneten Kontakte zur Kontaktierung eines Regelstromkreises, während die mit 37b und 38b bezeichneten Kontakte zur hochohmigen Messung des Potentials an den beiden Elektroden 17 und 18 dienen. Das zwischen diesen beiden "Fühlerkontakten" (sense contacts) gemessene Signal wird über einen hochohmigen Impedanzwandler 39 in die Regelung der zwischen den Elektroden anliegenden Spannung zurückgekoppelt. Dadurch werden Übergangswiderstände der Stromkontaktierungen 37a und 38a kompensiert.

Die zur Durchführung einer amperometrischen Analysemessung erforderliche Gleichspannungsquelle 40 wird von einem Digital-AnalogWandler gebildet, der entsprechend den Befehlen der Kontrolleinheit 34 ein DC-Signal erzeugt. Bei geschlossenem Schalter 41 liegt es an einem Spannungsfolger 42 an, dessen Ausgangsspannung aufgrund der Rückkopplung stets so hoch ist, daß die an den Fühlerkontakten 37b und, 38b gemessene Gleichspannung dem Sollwert entspricht. Das bei Anliegen dieser Spannung infolge der Reaktion in dem Biosensor fließende Stromsignal wird über eine insgesamt mit 43 bezeichnete DC-Strommeßeinrichtung gemessen, die einen Strom-Spannungswandler 44 und einen Analog-Digital-Wandler 45 umfaßt. Insgesamt bilden somit die DC-Spannungsquelle 40 (in Verbindung mit den Komponenten 39 und 42) und die DC-Strommeßeinrichtung 43 eine insgesamt mit 46 bezeichnete Analysemeßeinrichtung.

Zur Durchführung der Flächenkompensationsmessung weist die Elektronikeinheit 28 eine insgesamt mit 48 bezeichnete Widerstandsmeßeinrichtung auf, die mit Wechselstrom (AC) arbeitet. Die hierzu erforderliche Wechselspannungsquelle wird von einem AC-Generator 49 in Verbindung mit den zuvor erläuterten Komponenten 39 und 42 gebildet, an die er bei geschlossenem Schalter 50 angeschlossen ist. Auch die AC-Spannung wird mittels der 4-Kontakt-Technik so gesteuert, daß, unabhängig von Kontakt- und Leitungswiderständen, die gewünschte Sollspannung an den Elektroden 17,18 anliegt. Die für die AC-Widerstandsmessung erforderliche AC-Strommessung erfolgt mittels eine Meßwiderstandes 51, dessen Spannungsabfall mit einem Differenz-Meßverstärker 52 und einem nachgeschalteten Analog-Digital-Wandler 53 gemessen und in digitaler Form an die Kontroll- und Auswerteeinheit 34 weitergeleitet wird.

Der Wechselstromwiderstand (Impedanz) ist bekanntlich eine vektorielle Größe (mit einem Realteil und einem Imaginärteil), die beispielsweise dadurch bestimmt werden kann, daß man die an den Elektroden anliegende Spannung, den dabei fließenden Strom und die Phasenverschiebung zwischen Spannung und Strom mißt. Daraus kann man weitere gewünschte Werte (Realteil, Imaginärteil, Betrag der Impedanz) auf bekannte Weise berechnen. Näheren Einzelheiten sind beispielsweise der WO 99/32881 zu entnehmen.

Ein Wechselstromwiderstandsmeßwert im Sinne der Erfindung ist jeder bei einer Wechselstrom-Widerstandsmessung gewonnene Meßwert, also insbesondere der Betrag der Impedanz (bzw. deren Kehrwert, der Admittanz) und die Phasenverschiebung, wobei selbstverständlich weder auf die mathematische Beschreibung des Meßwertes (Realteil und/oder Imaginärteil; Betrag und/oder Phase) noch darauf ankommt, daß der Meßwert in gebräuchlichen Einheiten ausgedrückt wird. In der Praxis liegt der Meßwert in der mikroprozessorgesteuerten Auswerteelektronik des Gerätes in digitaler Form vor und wird in dieser Form weiterverarbeitet, ohne in übliche Einheiten konvertiert zu werden.

Durch Wechselstrom-Widerstandsmessungen bei mehreren Wechselstromfrequenzen (insbesondere in dem weiter oben angegebenen Frequenzbereich) lassen sich Meßfehler (insbesondere hinsichtlich der Flächenkompensationsmessung) eliminieren, die durch unterschiedliche Einflußfaktoren entstehen können. Insbesondere geht es um die Kompensation von Meßfehlern, die durch von Messung zu Messung variierende Konzentrationen korpuskulärer Bestandteile (d.h. im Falle von Blut Schwankungen des Hämatokritwertes) in der Probenflüssigkeit und/oder durch Temperaturschwankungen verursacht werden.

Sowohl durch die Temperatur als auch durch die Partikelkonzentration wird die Diffusion der Ladungsträger in der Probenflüssigkeit und damit ihre spezifische Leitfähigkeit beeinflußt. Derartige Leitfähigkeitsvariationen stören jedoch die Flächenkompensationsmessung, weil damit eine Unsicherheit besteht, ob eine Änderung des gemessenen Leitwertes durch eine Änderung der benetzten Teilfläche der effektiven Elektrodenstrukturfläche oder durch einen der genannten Störfaktoren verursacht ist. Soweit derartige Störungen zu einer nicht vertretbaren Einschränkung der Meßgenauigkeit führen, bestehen mehrere Möglichkeiten zur Elimination von damit verbundenen Meßfehlern:

- Der Störfaktor kann beseitigt werden. Im Falle der Temperaturstörung dient hierzu die Thermostatisierung des Testfeldes. Im Falle der Hämatokrit-Störung das Abfiltern der korpuskulären Bestandteile.

- Die Temperaturstörung kann durch Temperaturmessung und eine hierauf basierende Korrekturrechnung eliminiert werden.

- Auch ohne diese Maßnahmen kann auf der Grundlage von bei verschiedenen AC-Frequenzen gemessenen Wechselstrom-Widerstandsmeßwerten ("Impedanzspektroskopie") eine Elimination des Meßfehlers erreicht werden.

Bei dem letztgenannten Verfahren erfolgt die Auswertung vorzugsweise mittels numerisch-statistischer Methoden, beispielsweise der Multivariatenanalyse oder der Hauptkomponentenanalyse. Ergänzend können geeignete Modellannahmen gemacht werden. Auch zur Korrektur von Störeinflüssen durch Hämatokrit- und Temperaturvariation kann auf einschlägige Dokumente, insbesondere die erwähnte WO 99/32881 verwiesen werden.

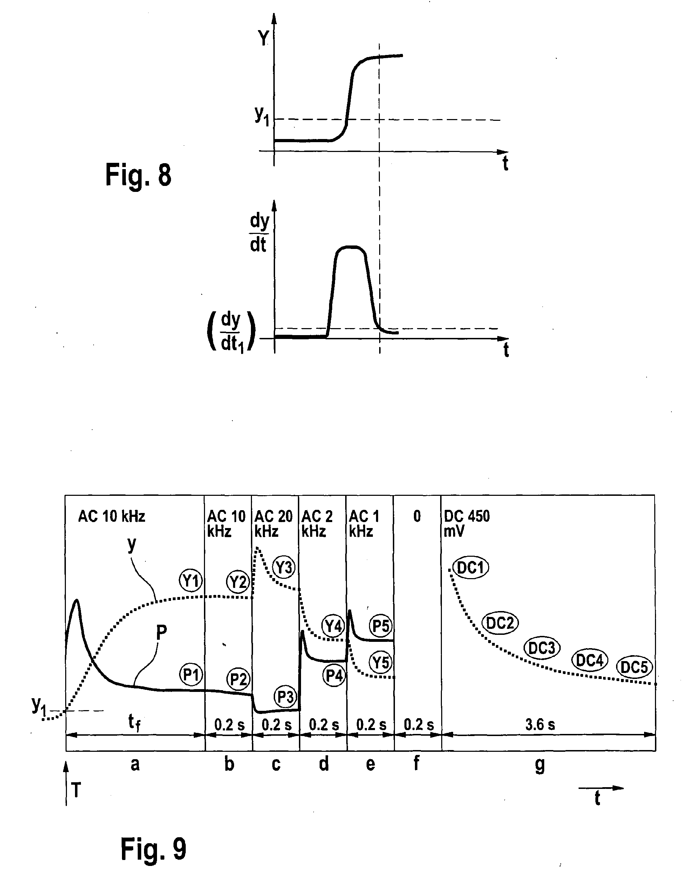

Der obere Teil von Figur 8 zeigt den Verlauf des Betrages der Admittanz Y gegen die Zeit t während der Aufgabe und des Eindringens eines Tropfens Probenflüssigkeit in das Testfeld, wobei dieser Prozeß in Figur 7 in drei Phasen symbolisch dargestellt ist. Das Auswertegerät wird (vorzugsweise durch Einschieben des Biosensors automatisch) eingeschaltet. Zugleich wird die Heizeinrichtung 12 eingeschaltet und auf eine erhöhte Temperatur (beispielsweise 37°C) eingestellt. Wenn diese Temperatur erreicht ist, ist das Gerät bereit zur Aufgabe der Probe. Sie erfolgt zweckmäßigerweise, wie in Figur 7 dargestellt, durch unmittelbare Übertragung von dem Körperteil (Finger), an dem der zu untersuchende Flüssigkeitstropfen durch einen Einstich gewonnen wurde. Sobald die Probenflüssigkeit in das Testfeld eindringt und die Elektrodenstruktur kontaktiert, steigt Y an. Durch Vergleich mit einem Schwellwert $Y_1$ wird die Probenaufgabe von dem Gerät erkannt. Die nachfolgende Ausbreitung der Probenflüssigkeit auf der effektiven Elektrodenstrukturfläche führt zu einem weiteren Anstieg des Leitwertes Y, bis schließlich die Ausbreitung nicht weiter fortschreitet. Dieser Zeitpunkt, zu dem der eigentliche Meßalgorithmus beginnen kann, läßt sich vorteilhaft dadurch ermitteln, daß man die zeitliche Änderung des Betrages der Admittanz dY/dt, die in der unteren Hälfte von Figur 8 dargestellt ist, verfolgt und diesbezüglich einen Schwellwert $(dY/dt)_1$ festlegt, zu dem die Admittanzänderung so weit abgenommen hat, daß der Meßalgorithmus ablaufen kann.

Figur 9 zeigt typische Meßsignale, die während des gesamten Detektions- und Meßalgorithmus gemessen werden. Dargestellt ist der Betrag der Admittanz Y und die Phase P einer Wechselstrom-Leitfähigkeitsmessung während fünf aufeinanderfolgenden Zeitabschnitten a bis e, sowie ein Zeitabschnitt f, während dessen keine Spannung an den Elektroden anliegt, und der gemessene DC-Strom in einem Zeitabschnitt g, in dem die DC-Analysemessung durchgeführt wird.

Zu dem mit dem Pfeil T symbolisierten Zeitpunkt erfolgt die Detektion der Probenaufgabe wie vorstehend beschrieben. Am Ende der mit $t_f$ bezeichneten Füllzeit hat die Admittanzänderung soweit abgenommen, daß der Meßalgorithmus beginnen kann. Während des Zeitraumes a läuft also der anhand von Figur 8 beschriebene Probendetektionsalgorithmus ab.

Der erste Teil des Meßalgorithmus besteht aus den Zeitabschnitten b bis e, während denen Y und P bei den angegebenen Frequenzen 10 kHz, 20 kHz, 2 kHz und 1 kHz jeweils für 0,2 Sekunden gemessen werden. Diese Messung bei einer Mehrzahl unterschiedlicher Wechselstromfrequenzen (die auch als Impedanzspektroskopie bezeichnet wird) ermöglicht wie erläutert die Elimination von Meßfehlern.

Nach einem anschließenden Zwischenzeitabschnitt f, währenddessen keine Spannung an den Elektroden anliegt, wird die für die Analysemessung erforderliche Gleichspannung (hier beispielsweise 450 mV) angelegt und es wird der dabei resultierende Gleichstrom (hier zu fünf aufeinander folgenden Meßzeitpunkten DC1 bis DC5) gemessen.

Im Rahmen des dargestellten Meßalgorithmus werden die AC-Messung und die DC-Messung (beispielsweise gesteuert durch die Schalter 41 und 50 in Figur 6) in engem zeitlichem Abstand nacheinander durchgeführt. Es ist aber auch eine Simultanmessung möglich, wenn die DC-und AC-Komponenten beider Messungen mit elektronischen Mitteln getrennt werden. Die AC-Messung dient - wie erläutert - zur Flächenkompensation. Ihr Ergebnis wird berücksichtigt, wenn aus der DC-Analysemessung von der Auswerteeinheit 34 das gewünschte analytische Ergebnis ermittelt wird.

Die Berücksichtigung des Wechselstromwiderstandsmeßwertes bei der Ermittlung des analytischen Ergebnisses läßt sich am Beispiel der Bestimmung einer gemessenen Konzentration C wie folgt erläutern:

- Bei gegebener Gleichspannung ist bei der Analysemessung gemessene Gleichstrom $I_{DC}$ proportional zu der benetzten Teilfläche A der effektiven Elektrodenstrukturfläche:

$$I_{DC} = k_1 \cdot A \cdot C$$

Darin ist C die Analytkonzentration in der Probe und $k_1$ eine Proportionalitätskonstante.

- Die benetzte Fläche A ist proportional zu dem Betrag der Admittanz, bei gegebener Wechselstromspannung also zu dem gemessenen Wechselstrom $I_{AC}$

$$A = k_2 \cdot I_{AC}$$

Darin ist $k_2$ eine Proportionalitätskonstante.

- Einsetzen folgt, daß sich die gesuchte Konzentration berechnen läßt gemäß

$$C = I_{DC} / (k_1 k_2 I_{AC})$$

Der gemessene Wechselstromwiderstandsmeßwert wird in diesem einfachen Beispiel also dadurch berücksichtigt, daß der im Rahmen der Analysemessung bestimmte DC-Stromwert durch den im Rahmen der Kompensationsmessung gemessenen Betrag des Wechselstromwiderstandes dividiert und dadurch die von Messung zu Messung unterschiedliche Größe der benetzten Fläche kompensiert wird.

In der Praxis erfolgt die Berücksichtigung des Wechselstromwiderstandsmeßwertes vorzugsweise im Rahmen eines numerischen Auswertealgorithmus, der außer der Größe der benetzten Teilfäche auch andere Einflußfaktoren, beispielsweise Temperatur und Hämatokrit sowie nichtlineare Zusammenhänge berücksichtigt. Dabei können beispielsweise sämtliche Meßvariablen ("observables"), also DC-Strommeßwerte, AC-Strommeßwerte und Phasenmeßwerte mit Gewichtungsfaktoren gewichtet und einer multivariaten Analyse unter Verwendung (rein empirischer oder auch phänomenologisch begründeter) funktionaler Zusammenhänge unterworfen werden. Bei einem solchen Verfahren werden mittels Messungen bei bekannten Konzentrationen Faktoren ermittelt, die die Auswertung unter Berücksichtigung sämtlicher im Einzelfall relevanter Einflußfaktoren, hier also insbesondere auch der benetzten Fläche A, ermöglichen. Da derartige Verfahren einerseits bekannt sind und andererseits in einer sehr großen Vielfalt von Varianten verwendet werden, ist eine nähere Beschreibung weder möglich noch notwendig. Entscheidend im Rahmen der vorliegenden Erfindung ist lediglich, daß ein Wechselstromwiderstandsmeßwert im Rahmen des Auswertealgorithmus als (indirektes) Maß für die Größe der benetzten Fläche A verwendet wird.

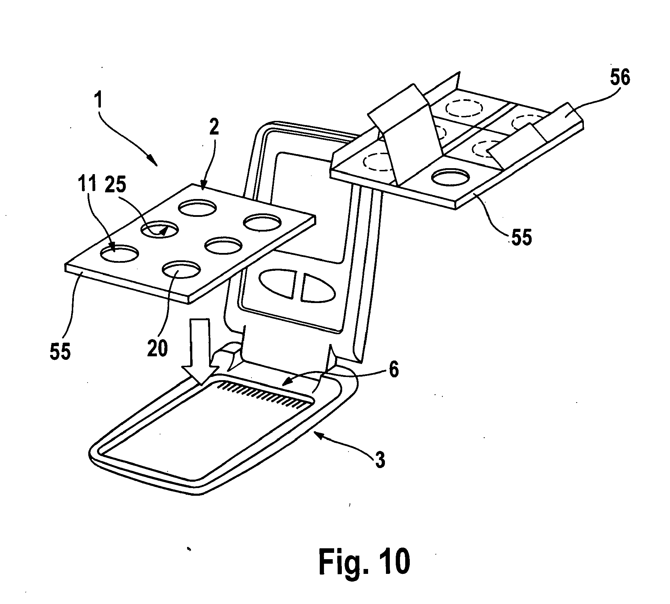

Die Figuren 10 bis 12 zeigen eine Ausführungsform eines Analysesystems 1, bei dem eine Mehrfach-Testkarte 55 verwendet wird, die mehrere (im dargestellten Fall sechs) Biosensoren 2 enthält. Das zugehörige Auswertegerät 3 hat eine Vielzahl von Gerätekontakten 6, die die erforderliche Verbindung zu den entsprechenden Sensorkontakten 5 sämtlicher Biosensoren 2 herstellen, wenn eine Mehrfach-Testkarte 55 in das Auswertegerät 3 eingesetzt wird. Die Probenaufgabeflächen 11 und die darunter befindlichen Testfelder 20 der Biosensoren 2 sind jeweils durch Schutzfolien 56 abgedeckt, die abgezogen werden können, bevor der jeweils darunter befindliche Biosensor benutzt wird.

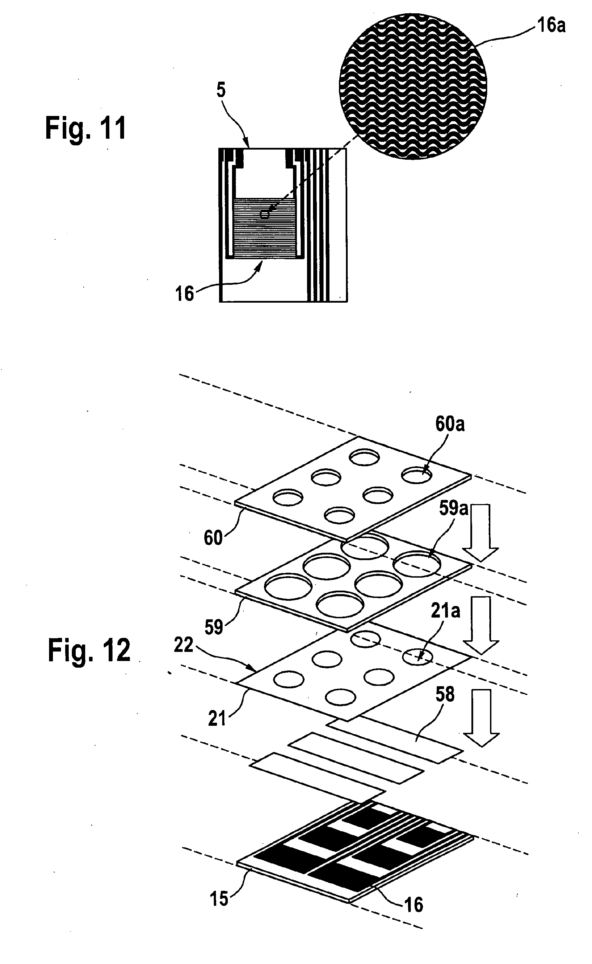

Figur 11 zeigt beispielhaft eine Elektrodenstruktur 16 eines Biosensors 2 einer Testkarte 55. Die dargestellte Ausschnittsvergrößerung verdeutlicht, daß die Leiterbahnen der Elektrodenstruktur 16 extrem fein sind.

Figur 12 zeigt einen bevorzugten mehrschichtigen Aufbau der Testkarte 55. Die auf einer Tragschicht 15 angeordneten Elektrodenstrukturen 16 der sechs Biosensoren werden von Streifen 58 eines Testfeldmaterials überdeckt. Die Testfelder 20 werden von einer Maskierungsschicht 22 begrenzt, die von einer Kunststoffolie 21 mit entsprechend ausgestanzten Ausnehmungen 21 a gebildet wird.

Darüber befindet sich eine optionale Distanzfolie 59, deren Ausnehmungen 59a größer sind als die Ausnehmungen der Kunststoffolie 21. Die Distanzfolie 59 ist wiederum von einer Abdeckschicht 60 bedeckt, deren Ausnehmungen 60a kleiner sind als die Ausnehmungen 59a der Distanzfolie 59. Durch diese Schichtenfolge und die erwähnten Größenverhältnisse der Ausnehmungen ergeben sich Kapillarkammern in Form eines Ringspaltes zwischen den Schichten 21 und 60, die dazu dienen, einen Überschuß an Probenflüssigkeit aufzunehmen, wenn die auf das Probenaufgabefeld 11 aufgegebene Flüssigkeitsmenge so groß ist, daß es bis an seinen Rand gefüllt wird. Durch diese Maßnahme wird der Anwendungsbereich des Biosensors auch auf Anwendungsfälle erweitert, bei denen so große Probenflüssigkeitsmengen vorkommen, daß die Probenaufgabefläche 11 nicht nur vollständig bedeckt wird, sondern ein darüber hinausgehender Überschuß vorhanden ist.

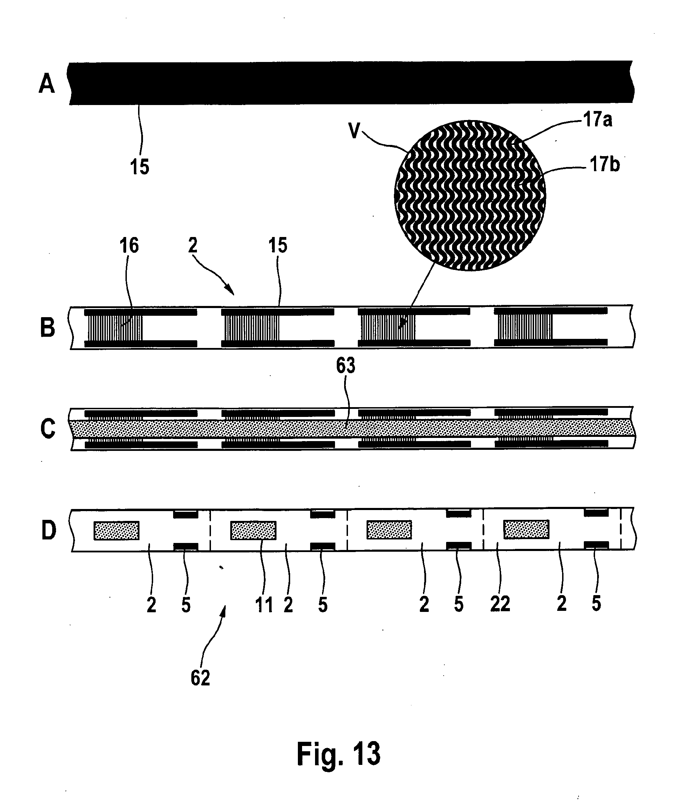

Figur 13 zeigt ein erfindungsgemäßes Sensorband 62 in vier Produktionsphasen A bis D, jeweils in schematischer Aufsicht. Die Produktionsphasen entsprechen den oben zu den Figuren 3 bis 5 gegebenen' Erläuterungen:

- In Phase A wird auf die Tragschicht 15 ein Elektrodenmaterial als leitende Beschichtung aufgetragen.

- In Phase B werden die nicht gewünschten Teile der leitenden Beschichtung, beispielsweise durch Laserablation, entfernt und dadurch die Elektrodenstrukturen 16 von Biosensoren 2 gebildet, die auf dem Trägerband 15 hintereinander angeordnet sind. Die mit V bezeichnete Vergrößerung verdeutlicht Einzelheiten der bevorzugten Elektrodenstruktur mit wellenförmigen Sekundärleitern 17a,17b.

- In Phase C erfolgt der Auftrag des hier mit 63 bezeichneten Testfeldmaterials, zweckmäßigerweise ununterbrochen in Längsrichtung der Tragschicht.

- Schließlich wird in Phase D eine Maskierungsschicht 22 aufgebracht, deren Fenster die Probenaufgabefläche 11 und die Sensorkontaktfläche 5 der Sensoren 2 des Sensorbandes 62 frei lassen.

Ein derartiges Sensorband kann vorteilhaft in einem Auswertegerät in Form einer aufgewickelten Spule vorrätig und mittels einer zweiten Spule aufgewickelt werden. Es wird schrittweise jeweils so transportiert, daß sich einer der Sensoren in einer Meßposition befindet, in der die Probe auf seine Probenaufgabefläche 11 aufgebracht wird. Dabei stehen die Sensorkontakte 5 mit entsprechenden Kontakten des Auswertegerätes, die beispielsweise als Schleifkontakte ausgebildet sein können, in elektrischer Verbindung. Nähere Erläuterungen sind nicht erforderlich, weil Sensorbänder bekannt sind. Die Erfindung ist in diesem Zusammenhang jedoch besonders vorteilhaft, weil sie einerseits eine kostengünstige und einfache Herstellung des Sensorbandes und andererseits eine einfache Handhabung durch den Benutzer ermöglicht.

**Patentansprüche**

1. Elektrochemisches Biosensor-Analysesystem (1) zur Analyse einer Probenflüssigkeit, insbesondere einer Körperflüssigkeit, umfassend

   a) einen zur einmaligen Verwendung vorgesehenen Biosensor (2) mit
   einer Tragschicht (15),
   einer auf der Tragschicht (15) angeordneten Elektrodenstruktur (16) mit mindestens zwei Elektroden (17,18)
   einem auf der Tragschicht (15) angeordneten, die Elektrodenstruktur (16) überdeckenden, zur Aufnahme der Probenflüssigkeit geeigneten Testfeld (20), das ein Reagenzsystem enthält, dessen Reaktion mit der Probenflüssigkeit zu einer Änderung einer elektrischen, für das gewünschte analytische Ergebnis charakteristischen Meßgröße führt, die mittels einer Analysemessung unter Verwendung von Elektroden der Elektrodenstruktur gemessen werden kann, und
   einer Probenaufgabefläche (11) auf der von der Tragschicht (15) abgewandten Oberseite des Testfeldes (20); und
   b) ein Auswertegerät (3) mit
   einer Analysemeßeinrichtung zum Messen der Meßgröße an jeweils einem in einer Meßposition positionierten und mit der Meßeinheit elektrisch verbundenen Biosensor (2) und
   einer Auswerteeinheit (34) zur Ermittlung der gewünschten Analysedaten aus gemessenen Werten der Meßgröße,

   **dadurch gekennzeichnet, daß**
   die Elektrodenstruktur auf einer von der Probenflüssigkeit benetzbaren effektiven Elektrodenstrukturfläche (23) hinsichtlich einer Wechselstrom-Widerstandsmessung an einer Flüssigkeitsschicht mit einer definierten Leitfähigkeit und Schichtdicke homogen verteilt ist,
   das Testfeld so ausgebildet ist, daß ein auf die Probenaufgabefläche (11) aufgegebener Tropfen Probenflüssigkeit sich mindestens über eine Teilfläche der Probenaufgabefläche (11) ausbreitet und eine entsprechende Teilfläche der effektiven Elektrodenstrukturfläche (23) benetzt wird, und
   das Auswertegerät eine Wechselstromwiderstandsmeßeinrichtung (48) aufweist, um einen Wechselstromwiderstandsmeßwert zwischen zwei Elektroden (17,18) der Elektrodenstruktur (16) als Flächenkompensationsmessung zu messen, wobei der gemessene Wechselstromwiderstandsmeßwert bei der Ermittlung der Analysedaten als Maß für die von der Probenflüssigkeit benetzte Teilfläche der effektiven Elektrodenstrukturfläche (23) verwendet wird.

2. Analysesystem nach Anspruch 1, **dadurch gekennzeichnet, daß** das Auswertegerät (3) eine Heizeinrichtung (14) zum Erwärmen des Testfeldes (20) eines in der Meßposition positionierten Biosensors (2) aufweist.

3. Analysesystem nach einem der Ansprüche 1 oder 2, **dadurch gekennzeichnet, daß** es eine Temperaturmeßeinrichtung (31,32) zur Messung der Temperatur des Testfeldes (20) eines in der Meßposition positionierten Biosensors (2) aufweist.

4. Analysesystem nach den Ansprüchen 2 und 3, **dadurch gekennzeichnet, daß** das Auswertegerät (3) eine Thermostatisierungselektronik aufweist, um das Testfeld (11) mittels der Temperaturmeßeinrichtung (31,32) und der Heizeinrichtung (12) auf eine gewünschte Solltemperatur zu thermostatisieren.

5. Analysesystem nach einem der vorhergehenden Ansprüche, **dadurch gekennzeichnet, daß** das Auswertegerät (3) Wechselstromwiderstandsmeßwerte dazu verwendet, Meßfehler zu eliminieren, die durch von Messung zu Messung variierende Konzentrationen korpuskulärer Bestandteile in der Probenflüssigkeit und/oder durch eine Änderung der Temperatur des Testfeldes (20) verursacht werden.

6. Analysesystem nach Anspruch 5, **dadurch gekennzeichnet, daß** bei der Messung der Wechselstromwiderstandsmeßwerte eine Mehrzahl von Wechselspannungen unterschiedlicher Frequenz an die Elektroden angelegt werden.

7. Analysesystem nach einem der vorhergehenden Ansprüche, **dadurch gekennzeichnet, daß** der Biosensor (2) als langgestreckter Teststreifen mit einer zum Einführen in eine Halterung (4) des Auswertegerätes (3) vorgesehenen Einführungsende (13), und einem dem Einführungsende (13) gegenüberliegenden Handhabungsende (14) ausgebildet ist, wobei sich das Testfeld (11) zwischen dem Einführungsende (13) und dem Handhabungsende (14) befindet.

8. Analysesystem nach einem der vorhergehenden Ansprüche, **dadurch gekennzeichnet, daß** ein in einer Halterung (4) des Auswertegerätes (3) positionierter Biosensor (2) von einem gegenüber den benachbarten Bauteilen des Auswertegerätes (3) erhabenen Biosensorlager (10) unterstützt wird.

9. Analysesystem nach einem der vorhergehenden Ansprüche, **dadurch gekennzeichnet, daß** der Biosensor Bestandteil eines aus einer Mehrzahl von miteinander verbundenen Biosensoren bestehenden Biosensorbandes (62) ist.

10. Biosensor für ein Analysesystem nach einem der vorhergehenden Ansprüche, mit einer Tragschicht (15), einer auf der Tragschicht (15) angeordneten Elektrodenstruktur (16) mit mindestens zwei Elektroden (17,18), einem auf der Tragschicht (15) angeordneten, die Elektrodenstruktur (16) überdeckenden, zur Aufnahme der Probenflüssigkeit geeigneten Testfeld (20), das ein Reagenzsystem enthält, dessen Reaktion mit der Probenflüssigkeit zu einer Änderung einer elektrischen, für das gewünschte analytische Ergebnis charakteristischen Meßgröße führt, die mittels einer Analysemessung unter Verwendung von Elektroden der Elektrodenstruktur gemessen werden kann, und einer Probenaufgabefläche (11) auf der von der Tragschicht (15) abgewandten Oberseite des Testfeldes (20), **dadurch gekennzeichnet, daß** zwei Elektroden (17,18) der Elektrodenstruktur (16) jeweils kammartig mit einem Primärleiter (17a,18a) und davon abzweigenden quer zu der Richtung des Primärleiters (17a,18a) verlaufenden Sekundärleitern (17b,18b) ausgebildet sind und die Sekundärleiter (17b,18b) alternierend ineinander greifen.

11. Biosensor nach Anspruch 10, **dadurch gekennzeichnet, daß** sich die Richtung, in der die Sekundärleiter (17b,18b) verlaufen, über deren Länge mehrfach ändert.

12. Biosensor nach Anspruch 10, **dadurch gekennzeichnet, daß** die Probenaufgabefläche (12) durch eine Maskierungsschicht (22) begrenzt ist.

13. Biosensor nach einem der Ansprüche 10 bis 12, **dadurch gekennzeichnet, daß** das Material des Testfeldes (20) derartig quellfähig ist, daß sich das Volumen des Testfeldes (20) bei der Aufnahme der Probenflüssigkeit vergrößert.

14. Biosensor nach einem der Ansprüche 10 bis 13, **dadurch gekennzeichnet, daß** das Testfeld Separationsmittel zur Abtrennung korpuskulärer Bestandteile aus der Probenflüssigkeit enthält, welche so ausgebildet sind, daß im wesentlichen nur Plasma zu der Elektrodenstruktur (10) gelangt und diese benetzt.

15. Biosensor nach einem der Ansprüche 10 bis 14, **dadurch gekennzeichnet, daß** er eine der Probenaufgabefläche (11) benachbarte Kapillarkammer (25,26) zur Aufnahme eines Überschußvolumens der Probenflüssigkeit aufweist.

**16.** Verfahren zur Analyse einer Probenflüssigkeit, insbesondere einer Körperflüssigkeit,
mittels eines Biosensor-Analysesystems, umfassend

a) einen zur einmaligen Verwendung vorgesehenen Biosensor (2) mit
einer Tragschicht (15),
einer auf der Tragschicht (15) angeordneten Elektrodenstruktur (16) mit mindestens zwei Elektroden (17,18)
einem auf der Tragschicht (15) angeordneten, die Elektrodenstruktur (16) überdeckenden, zur Aufnahme der Probenflüssigkeit geeigneten Testfeld (20), das ein Reagenzsystem enthält, dessen Reaktion mit der Probenflüssigkeit zu einer Änderung einer elektrischen, für das gewünschte analytische Ergebnis charakteristischen Meßgröße führt, die unter Verwendung von Elektroden der Elektrodenstruktur gemessen werden kann, und
einer Probenaufgabefläche (11), auf der von der Tragschicht (15) abgewandten Oberseite des Testfeldes (20), und
wobei die Elektrodenstruktur auf einer von der Probenflüssigkeit benetzbaren effektiven Elektrodenstrukturfläche (23) hinsichtlich einer Widerstandsmessung an einer Flüssigkeitsschicht mit einer definierten Leitfähigkeit und Schichtdicke homogen verteilt ist,
b) ein Auswertegerät (3) mit
einer Analysemeßeinrichtung zum Messen der Meßgröße an jeweils einem in einer Meßposition positionierten und mit der Meßeinheit elektrisch verbundenen Biosensor (2) und und
einer Auswerteeinheit (34) zur Ermittlung der gewünschten Analysedaten aus gemessenen Werten der Meßgröße,

bei welchem
ein Tropfen der Probe von oben derartig mit der Probenaufgabefläche (11) in Kontakt gebracht wird, daß er sich mindestens über eine Teilfläche der Probenaufgabefläche (11) ausbreitet und eine entsprechende Teilfläche der effektiven Elektrodenstrukturfläche (23) benetzt wird,
innerhalb einer Zeitspanne, in der sich die Größe der benetzten Fläche nicht wesentlich ändert, folgende Messungen durchgeführt werden:

a) eine Analysemessung, bei der mittels der Elektroden die elektrische, für das gewünschte analytische Ergebnis charakteristische Meßgröße gemessen wird und
b) eine Flächenkompensationsmessung, bei der zwischen zwei Elektroden (17,18) der Elektrodenstruktur (16) ein Wechselstromwiderstandsmeßwert gemessen wird, und

der gemessene Wechselstromwiderstartdsmeßwert bei der Ermittlung der Analysedaten als Maß für die von der Probenflüssigkeit benetzte Teilfläche der effektiven Elektrodenstrukturfläche (23) verwendet wird.

Fig. 1

Fig. 2

14

**Fig. 3**

**Fig. 4**

**Fig. 5**

**Fig. 6**

**Fig. 7**

Fig. 8

Fig. 9

**Fig. 10**

**Fig. 11**

**Fig. 12**

Fig. 13

**Europäisches Patentamt**

# EUROPÄISCHER RECHERCHENBERICHT

Nummer der Anmeldung

EP 06 00 1499

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2005/023154 A1 (KERMANI MAHYAR Z ET AL) 3. Februar 2005 (2005-02-03) * Zusammenfassung; Abbildungen 1-3 * * Absätze [0007] - [0021], [0048] - [0086] * ----- | 1-16 | INV. G01N27/30 G01N33/487 G01N27/04 C12Q1/00 |
| Y | US 5 395 504 A (SAURER ET AL) 7. März 1995 (1995-03-07) * Spalten 5-7; Abbildungen 1-14 * ----- | 1,5-7,9, 13,16 | |
| Y | PATENT ABSTRACTS OF JAPAN Bd. 011, Nr. 162 (P-579), 26. Mai 1987 (1987-05-26) & JP 61 294351 A (MATSUSHITA ELECTRIC IND CO LTD), 25. Dezember 1986 (1986-12-25) * Zusammenfassung * ----- | 1,7,12, 14,16 | |
| Y | WO 85/02257 A (SENTECH MEDICAL CORPORATION) 23. Mai 1985 (1985-05-23) * Seite 19, Zeile 27 - Seite 20, Zeile 15; Abbildungen 1-3 * ----- | 2-4,8 | |
| Y | US 2001/045355 A1 (GEPHART CHAD STEPHEN ET AL) 29. November 2001 (2001-11-29) * Absatz [0050]; Abbildung 7 * ----- | 15 | RECHERCHIERTE SACHGEBIETE (IPC) G01N C12Q |
| Y | EP 1 311 702 A (DIABETES DIAGNOSTICS, INC) 21. Mai 2003 (2003-05-21) * Abbildungen 5-8 * ----- | 9 | |
| Y | EP 1 211 321 A (ROCHE DIAGNOSTICS GMBH; F. HOFFMANN-LA ROCHE AG) 5. Juni 2002 (2002-06-05) * Abbildungen 1,3 * ----- | 10,11 | |
| A | US 2004/256248 A1 (BURKE DAVID W ET AL) 23. Dezember 2004 (2004-12-23) * das ganze Dokument * ----- | 1-16 | |

-/--

Der vorliegende Recherchenbericht wurde für alle Patentansprüche erstellt

| Recherchenort | Abschlußdatum der Recherche | Prüfer |
|---|---|---|
| München | 8. Juni 2006 | Klein, M-0 |

**Europäisches**
**Patentamt**

**EUROPÄISCHER RECHERCHENBERICHT**

**Nummer der Anmeldung**

EP 06 00 1499

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 1 582 864 A (ROCHE DIAGNOSTICS OPERATIONS, INC) 5. Oktober 2005 (2005-10-05) * das ganze Dokument * ----- | 1-16 | |
| A | WO 2004/113915 A (ROCHE DIAGNOSTICS GMBH; F. HOFFMANN-LA ROCHE AG; GROLL, HENNING) 29. Dezember 2004 (2004-12-29) * das ganze Dokument * ----- | 1-16 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 8. Juni 2006 | Klein, M-O |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

    ...............................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 06 00 1499

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-06-2006

| Im Recherchenbericht angeführtes Patentdokument | Datum der Veröffentlichung | Mitglied(er) der Patentfamilie | Datum der Veröffentlichung |
|---|---|---|---|
| US 2005023154 A1 | 03-02-2005 | CA 2407249 A1<br>CN 1412548 A<br>CZ 20023368 A3<br>JP 2003185615 A<br>MX PA02010106 A<br>PL 356578 A1<br>TW 581866 B<br>US 2003098233 A1 | 10-04-2003<br>23-04-2003<br>18-06-2003<br>03-07-2003<br>14-10-2004<br>22-04-2003<br>01-04-2004<br>29-05-2003 |
| US 5395504 A | 07-03-1995 | AU 667152 B2<br>AU 5487394 A<br>CA 2114685 A1<br>DE 69411963 D1<br>DE 69411963 T2<br>DK 609760 T3<br>EP 0609760 A1<br>ES 2122049 T3<br>FR 2701117 A1<br>JP 3488501 B2<br>JP 6294769 A | 07-03-1996<br>11-08-1994<br>05-08-1994<br>03-09-1998<br>12-05-1999<br>26-04-1999<br>10-08-1994<br>16-12-1998<br>05-08-1994<br>19-01-2004<br>21-10-1994 |
| JP 61294351 A | 25-12-1986 | JP 6048256 B | 22-06-1994 |
| WO 8502257 A | 23-05-1985 | EP 0163694 A1<br>JP 61500508 T | 11-12-1985<br>20-03-1986 |
| US 2001045355 A1 | 29-11-2001 | KEINE | |
| EP 1311702 A | 21-05-2003 | AT 311472 T<br>AU 4960101 A<br>CA 2403646 A1<br>CN 1439057 A<br>DE 60115462 D1<br>DK 1311702 T3<br>ES 2252212 T3<br>JP 2003529061 T<br>MX PA02009666 A<br>PL 365243 A1<br>WO 0173109 A2<br>US 2003024811 A1<br>US 2004026243 A1 | 15-12-2005<br>08-10-2001<br>04-10-2001<br>27-08-2003<br>05-01-2006<br>27-03-2006<br>16-05-2006<br>30-09-2003<br>30-07-2004<br>27-12-2004<br>04-10-2001<br>06-02-2003<br>12-02-2004 |
| EP 1211321 A | 05-06-2002 | JP 3672525 B2<br>JP 2002214188 A<br>US 2002100684 A1 | 20-07-2005<br>31-07-2002<br>01-08-2002 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 06 00 1499

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-06-2006

| Im Recherchenbericht angeführtes Patentdokument | Datum der Veröffentlichung | Mitglied(er) der Patentfamilie | Datum der Veröffentlichung |
|---|---|---|---|
| US 2004256248 A1 | 23-12-2004 | CA 2530052 A1<br>EP 1639355 A1<br>WO 2005008231 A1 | 27-01-2005<br>29-03-2006<br>27-01-2005 |
| EP 1582864 A | 05-10-2005 | DE 9422380 U1 | 14-12-2000 |
| WO 2004113915 A | 29-12-2004 | CA 2529301 A1<br>CA 2529302 A1<br>EP 1639366 A1<br>EP 1642126 A1<br>WO 2005001474 A1<br>WO 2004113909 A1<br>WO 2004113911 A1<br>WO 2005003751 A1<br>WO 2004113914 A1 | 29-12-2004<br>29-12-2004<br>29-03-2006<br>05-04-2006<br>06-01-2005<br>29-12-2004<br>29-12-2004<br>13-01-2005<br>29-12-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5243516 A
- EP 0170375 A2
- US 5120420 A
- US 6645359 B
- WO 2004068138 A
- US 6880968 B
- WO 2004090533 A
- WO 2004030822 A1
- EP 0170375 A
- WO 9932881 A